(19) Europäisches Patentamt
European Patent Office
Office européen des brevets

(11) Veröffentlichungsnummer: **0 012 933**
**B1**

(12) # EUROPÄISCHE PATENTSCHRIFT

(45) Veröffentlichungstag der Patentschrift: **12.01.83**

(51) Int. Cl.³: **G 03 C 7/20,** G 03 C 1/00, G 03 C 7/32

(21) Anmeldenummer: **79105103.0**

(22) Anmeldetag: **12.12.79**

(54) **Fotografisches Aufzeichnungsmaterial.**

(30) Priorität: **23.12.78 DE 2855997**

(43) Veröffentlichungstag der Anmeldung: **09.07.80 Patentblatt 80/14**

(45) Bekanntmachung des Hinweises auf die Patenterteilung: **12.01.83 Patentblatt 83/2**

(84) Benannte Vertragsstaaten: **BE CH DE FR GB**

(56) Entgegenhaltungen:
**DE-A-2 646 750**
**FR-A-2 046 068**
**FR-A-2 287 057**
**US-A-3 671 259**

**RESEARCH DISCLOSURE, Nr. 143, März 1976, disclosure 14315 Havants, Hants "Method of incorporating colour photographic additives in hydrophilic colloids"**

(73) Patentinhaber: **AGFA-GEVAERT Aktiengesellschaft, D-5090 Leverkusen 1 (DE)**

(72) Erfinder: **Püschel, Walter, Dr., Berta-von-Suttner-Strasse 54, D-5090 Leverkusen 1 (DE)**
Erfinder: **Odenwälder, Heinrich, Dr., Am Arenzberg 37, D-5090 Leverkusen 31 (DE)**
Erfinder: **Ranz, Erwin, Dr., Euckenstrasse 1, D-5090 Leverkusen 1 (DE)**

**EP 0 012 933 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Fotografisches Aufzeichnungsmaterial

Die Erfindung betrifft ein fotografisches Aufzeichnungsmaterial mit mindestens einer lichtempfindlichen Silberhalogenidemulsionsschicht und mindestens einer Schicht, die eine nichtdiffundierende Verbindung enthält, die unter den Bedingungen der alkalischen Entwicklung eine diffundierende heterocyclische Mercaptoverbindung als Entwicklungsinhibitor freizusetzen vermag, das mindestens eine weitere Verbindung enthält, die Entwicklungsinhibitoren zu binden vermag.

Es ist bekannt, im Rahmen fotografischer Verfahren sogenannte Entwicklungsinhibitoren freisetzende Verbindungen zu verwenden, um die Entwicklung fotografischer Silberhalogenidemulsionsschichten selektiv zu steuern. Die Verwendung von Entwicklungsinhibitoren freisetzenden Verbindungen kann zu wesentlichen Verbesserungen der sensitometrischen Eigenschaften der fotografischen Materialien sowie der Bildstruktur durch Verminderung des Kontrastes und Ausbildung spezieller Entwicklungseffekte führen. In diesem Zusammenhang ist hinzuweisen auf den Artikel «Development-Inhibitor-Releasing (DIR) Couplers in Color Photography» in Photographic Science and Engineering Band 13, Seite 74 (1969). Die Verwendbarkeit von Entwicklungsinhibitoren freisetzenden Kupplern – im folgende DIR-Kuppler genannt –, die mit den Oxidationsprodukten von Farbentwicklerverbindungen zu reagieren vermögen und dabei einen Entwicklungsinhibitor freisetzen, ist beispielsweise bekannt aus der US-A-3 148 062. Aus der US-A-3 379 529 ist des weiteren beispielsweise die Verwendung von bestimmten Entwicklerverbindungen bekannt, die als Folge der Entwicklung des Silberhalogenides Entwicklungsinhibitoren freisetzen (DIR-Entwickler). Weitere Entwicklungsinhibitoren freisetzende Verbindungen, die Entwicklungsinhibitoren in anionischer Form bildweise freizusetzen vermögen, sind beispielsweise in den US-A-3 297 445, 3 227 554, 3 617 291 und 3 632 345 beschrieben. Durch Freisetzen dieser Inhibitoren bei der Entwicklung lässt sich die Körnigkeit vermindern, die Schärfe des zu entwikkelnden Bildes verbessern sowie die Gradation und Empfindlichkeit einer Silberhalogenidemulsion steuern. Weiterhin ist bekannt, einem fotografischen Material Substanzen zuzusetzen, aus denen unter den Bedingungen der fotografischen Entwicklung Entwicklungsinhibitoren in nicht bildmässiger Verteilung frei werden, was sich vielfach vorteilhaft auf den Schleier auswirkt. Hier ist beispielsweise die US-A-3 819 379 zu erwähnen, wonach der Inhibitor in komplexierter Form zugesetzt wird, oder die US-A-3 615 617, wonach der Inhibitor als Derivat mit blockierter Mercaptogruppe eingesetzt wird.

Oft ist es erwünscht, die Wirksamkeit der Inhibitoren unabhängig davon, ob sie in bildmässiger oder gleichförmiger Verteilung freigesetzt werden, räumlich zu beschränken, um zu verhindern, dass sie in andere Schichten als die, in der sie frei wurden, gelangen und dort in unerwünschter Weise die Entwicklung beinträchtigen oder gar aus dem Material heraus in die Entwicklerlösung, was sich in nachteiliger Weise auf nachfolgende Entwicklungsergebnisse auswirken müsste. Zu diesem Zweck ist es bekannt, in fotografischen Aufzeichnungsmaterialien Schichten vorzusehen, die Entwicklungsinhibitoren zu binden vermögen. Derartige «Abfangschichten» für Entwicklungsinhibitoren sind beispielsweise aus der GB-A-1 201 110 bekannt. In dieser Patentschrift ist beispielsweise ein farbfotografisches Aufzeichnungsmaterial beschrieben, das zwischen zwei Silberhalogenidemulsionsschichten üblicher Empfindlichkeit eine Zwischenschicht mit Silberhalogenidkörnern vergleichsweiser geringer Lichtempfindlichkeit enthält. Eine der genannten Silberhalogenidemulsionsschichten enthält einen Blaugrün-Kuppler mit einem Brom- oder Iod-Atom in der Kupplungsstelle, aus dem bei der Farbentwicklung Bromid oder Iodid-Ionen als Entwicklungsinhibitor in Freiheit gesetzt werden. Durch die Zwischenschicht vergleichsweise geringer Lichtempfindlichkeit werden die diffundierenden Entwicklungsinhibitoren adsorptiv gebunden und so die anderen Emulsionsschichten vor den Entwicklungsinhibitoren oder Entwicklungsinhibitor-Fragmenten geschützt. Aus der US-A-3 737 317 ist es weiterhin bekannt, Lippmann-Emulsionen als Deck- oder Trennschichten, und zwar entweder über, zwischen oder unter bilderzeugenden Silberhalogenidschichten anzuordnen, um hierdurch zu verhindern, dass Entwicklungsinhibitoren zwischen den Schichten wandern oder aus dem Aufzeichnungsmaterial in die Entwicklerlösung gelangen. Nachteilig an der Verwendung derartiger Deck- oder Trennschichten mit feinkörnigem Silberhalogenid ist jedoch, dass diese Schichten oftmals die sensitometrischen Eigenschaften der bilderzeugenden Schicht verändern. Eine solche Veränderung ist insbesondere dann unerwünscht, wenn die Lippmann-Emulsion als Fängerschicht für Entwicklungsinhibitor zwischen zwei bilderzeugenden Schichten verwendet wird, insbesondere in feinkörnigen Aufzeichnungsmaterialien. Aus der DE-A-2 448 232 ist des weiteren die Verwendung von Inhibitoren absorbierenden Kolloidschichten bekannt, die ein Polymer enthalten, das Entwicklungsinhibitoren zu absorbieren vermag. Alle die genannten Mittel zur Bindung von diffundierendem Entwicklungsinhibitor sind im alkalischen pH-Bereich der Entwicklerbäder wirksam, d.h. unter Bedingungen, bei denen üblicherweise der Entwicklungsinhibitor bildmässig oder in gleichförmiger Verteilung in Freiheit gesetzt wird. Die Wirkung der bekannten Inhibitorfänger ist damit an sich derjenigen der inhibitorfreisetzenden Verbindungen entgegengerichtet.

Bei Einsatz von Inhibitor freisetzenden Verbindungen, insbesondere DIR-Kupplern oder DIR-Entwicklern, können Schwierigkeiten dadurch auftreten, dass sie von der Synthese her Spuren freien Inhibitors enthalten, die unter technischen Synthesebedingungen nur schwer zu entfernen sind. Zum Teil sind solche Verbindungen unter den Lagerbedingungen der fertigen fotografischen Materialien nicht völlig stabil. Es treten Spuren von freien Inhibitoren auf, die die sensitometrischen Eigenschaften der Schichten in unerwünschtem Sinne beeinflussen können. Die zuvor beschriebenen Mittel zur Bindung von freiem Inhibitor sind nicht geeignet, diesen Nachteil zu beheben, da sie im alkalischen Bereich wirksam sind, d.h. unter Bedingungen, bei denen das Auftreten von freiem Inhibitor geradezu erwünscht ist.

Der Erfindung liegt die Aufgabe zugrunde, Massnahmen anzugeben, mit deren Hilfe ungewollt auftretende Spuren von freiem Inhibitor unwirksam gemacht werden können, die aber die bei der späteren Verarbeitung gewünschte Wirksamkeit der DIR-Verbindungen nicht beeinträchtigen.

Es wurde gefunden, dass durch die Verwendung von Verbindungen, die im schwach sauren oder neutralen Gebiet (pH kleiner als 7) Inhibitoren anzulagern vermögen, nicht aber oder jedenfalls sehr viel langsamer bei den höheren pH-Werten der fotografischen Entwicklung (pH 9—13), eine Schutzwirkung erzielt wird, wenn diese Verbindungen den Schichten zugesetzt werden, die die Inhibitoren freisetzenden Verbindungen enthalten. Es wird angenommen, dass diese Verbindungen mit den Gruppierungen der Inhibitoren, die für die Inhibitorwirkung verantwortlich sind, zu reagieren vermögen und diese blockieren. Als Inhibitoren gelangen heterocyclische Mercaptoverbindungen zum Einsatz, insbesondere solche, die eine Mercaptogruppe in Nachbarschaft zu einem Ringstickstoffatom enthalten.

Gegenstand der vorliegenden Erfindung ist ein fotografisches Aufzeichnungsmaterial mit mindestens einer lichtempfindlichen Silberhalogenidemulsionsschicht und mindestens einer Schicht, die eine nichtdiffundierende Verbindung enthält, die unter den Bedingungen der alkalischen Entwicklung eine diffundierende heterocyclische Mercaptoverbindung als Entwicklungsinhibitor in Freiheit zu setzen vermag. Das fotografische Aufzeichnungsmaterial ist dadurch gekennzeichnet, dass die gleiche Schicht, die die nichtdiffundierende, einen Entwicklungsinhibitor freisetzende Verbindung enthält, eine weitere Verbindung enthält, die bei pH-Werten kleiner als 7, nicht aber oder nur sehr viel weniger bei pH-Werten zwischen 9 und 13, freien Entwicklungsinhibitor zu binden vermag.

Für diesen Zweck geeignete Verbindungen entsprechen beispielsweise der folgenden allgemeinen Formel I

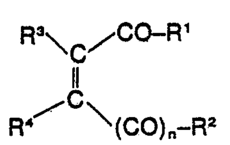

I

worin bedeuten

$R^1$ und $R^2$ (gleich oder verschieden) Wasserstoff, Alkyl, z.B. Methyl, Isobutyl, Aryl, z.B. Phenyl, Hydroxyl, Alkoxy, -NR-Alkyl oder -NR-Aryl, wobei R für Wasserstoff oder Alkyl steht oder beide Substituenten $R^1$ und $R^2$ zusammen den Rest zur Vervollständigung eines 5- oder 6gliedrigen carbocyclischen oder heterocyclischen Ringes, z.B. eines p-Chinonringes, eines γ-Pyronringes mit einem ankondensierten Ring oder eines Maleinimidringes, der am Stickstoffatom substituiert ist,

$R^3$ und $R^4$ (gleich oder verschieden) Wasserstoff, Alkyl mit bis zu 18 C-Atomen, Alkoxy, Aryl, Halogen wie Chlor, Alkoxycarbonyl oder Cyan

$n = 0$ oder 1, vorzugsweise 1, falls $R^1$ und $R^2$ einen Ring vervollständigen.

Die dargestellte Formel I bedeutet nicht notwendigerweise, dass die genannten Verbindungen in der cis-Konfiguration vorliegen müssen. Im Falle der nichtcyclischen Verbindungen ist in der Regel sogar anzunehmen, dass die Verbindungen überwiegend oder gar vollständig als trans-Isomere vorliegen.

Vorzugsweise enthalten die Verbindungen in einem der Reste $R^1$, $R^2$, $R^3$ und $R^4$ oder in einem durch $R^1$ und $R^2$ gebildeten Ring einen diffusionsfestmachenden Rest. Als diffusionsfestmachende Reste sind solche Reste anzusehen, die es ermöglichen die erfindungsgemässen Verbindungen in den üblicherweise bei fotografischen Materialien verwendeten hydrophilen Kolloiden diffusionsfest einzulagern. Hierzu sind vorzugsweise organische Reste geeignet, die im allgemeinen gradkettige oder verzweigte aliphatischen Gruppen und gegebenenfalls auch carbocyclische oder heterocyclische, gegebenenfalls aromatische Gruppen mit im allgemeinen 8 bis 20 C-Atomen enthalten. Mit dem übrigen Molekülteil sind diese Reste entweder direkt oder indirekt, z.B. über eine der folgenden Gruppen verbunden: $-NHCO-$, $-NHSO_2-$, $-NR-$, wobei R Wasserstoff oder Alkyl bedeutet, $-O-$ oder $-S-$. Zusätzlich kann der diffusionsfestmachende Rest auch wasserlöslichmachende Gruppen enthalten, wie z.B. Sulfogruppen oder Carboxylgruppen, die auch in anionischer Form vorliegen können. Da die Diffusionseigenschaften von der Molekülgrösse der verwendeten Gesamtverbindung abhängen, genügt es in bestimmten Fällen, z.B. wenn die erfindungsgemässe Inhibitorfängerverbindung unter Verwendung sogenannter Ölbildner oder hochsiedender Kupplerlösungsmittel in emulgierter Form in die Schicht eingearbeitet wird, als diffusionsfestmachende Reste auch kürzerkettige Reste, z.B. tert.-Butyl-, Isoamyl- oder tert.-Amylreste zu verwenden. Eine absolute Diffusionsfestigkeit der erfindungsgemässen Verbindungen ist im vorliegenden Fall jedoch nicht erforderlich, da eine nachteilige Wirkung dieser Verbindungen auf andere Schichten des fotografischen Materials nicht zu erwarten ist. Es genügt vielmehr, dass die Diffusionsfähigkeit der erfindungsgemässen Verbindung soweit eingeschränkt ist, dass nach der üblichen Lagerzeit des fotografischen Materials die Menge an erfindungsgemässer Verbindung in der ursprünglichen Schicht noch gross genug ist, um

dort unter Lagerbedingungen frei werdenden Inhibitor unwirksam zu machen.

Die erfindungsgemässen Verbindungen umfassen auch Polymere mit Struktureinheiten der angegebenen Formel I, die über einen der vorhandenen Substituenten, vorzugsweise über $R^1$ an ein vorgegebenes Polymergerüst geknüpft sind. Als geeignete Polymergerüste kommen beispielsweise Copolymere von Maleinsäure- oder Itaconsäureanhydrid mit copolymerisierbaren Monomeren wie Vinylmethylether oder Styrol in Frage, oder aber auch Copolymere von N-oxoalkylacrylamiden.

Beispiele für geeignete Verbindungen gemäss Erfindung sind im folgenden angegeben:

1. 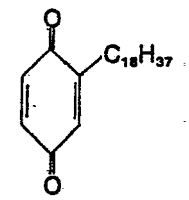

2. 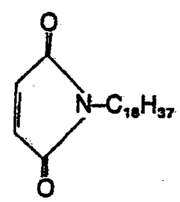

3. 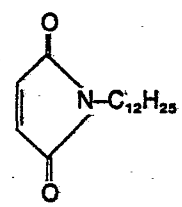

4. 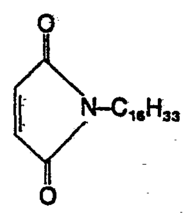

5. 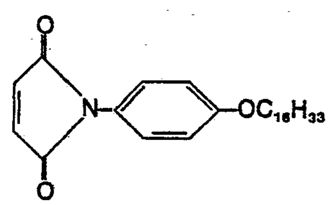

6. 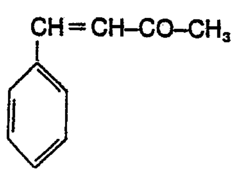

7. 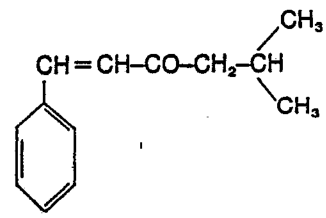

8. 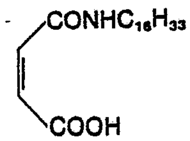

9. 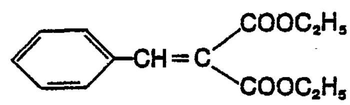

10. 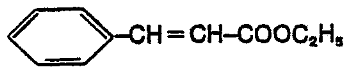

11. 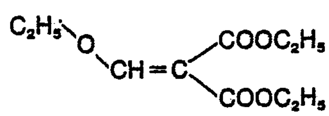

12. 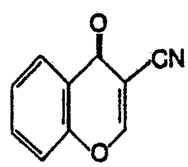

13. 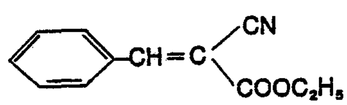

14. 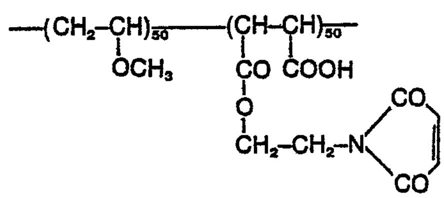

15. 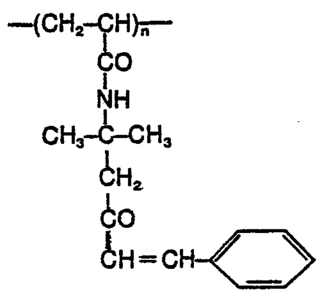

Die Herstellung der erfindungsgemässen Verbindungen ist einfach und erfolgt nach bekannten Methoden. Beispielhaft sei im folgenden die Herstellung einiger der erfindungsgemässen Verbindungen angegeben.

Herstellung der Verbindung 1

Zu 12 g Octadecylhydrochinon, die in 160 ml Eisessig suspendiert sind, werden unter Rühren portionsweise 3,3 g Natriumdichromat zugegeben. Nach 1,5 Stunden werden 300 ml Wasser zugegeben. Der entstandene Niederschlag wird abgesaugt, in 70 ml Chloroform gelöst. Nach Zugabe von Bleicherde wird filtriert und im Filtrat durch Zugabe von 200 ml Methanol das Octadecylbenzochinon unter Kühlung ausgefällt. Es werden 8,5 g vom Schmelzpunkt 94 bis 95 °C erhalten.

Herstellung der Verbindung 4

1. Stufe

19,6 g Maleinsäureanhydrid werden in 500 ml Toluol bei 90 °C gelöst. Bei Aufrechterhaltung einer Temperatur von 90 °C werden innerhalb 45 Minuten 48,2 g Hexadecylamin, gelöst in 200 ml Toluol, zugetropft. Nach einer weiteren Stunde Rühren bei 90 °C und Kühlung wird der entstandene Niederschlag abgesaugt und mit Methanol gewaschen. Es werden 44,5 g N-Hexadecylmaleinamid vom Schmelzpunkt 101 bis 102,5 °C erhalten.

2. Stufe

16,95 g der in der 1. Stufe erhaltenen Verbindung werden mit 2,05 g Natriumacetat in 20 ml Essigsäureanhydrid 30 Minuten unter Rückfluss erhitzt. Der Ansatz wird in 100 ml Wasser gegossen, der halbfeste Niederschlag in Chloroform aufgenommen, die Chloroformlösung mit Bleicherde verrührt, danach filtriert und das Filtrat eingeengt. Der Rückstand wird mit 400 ml 90%igem Methanol verrührt, abgesaugt, und mit 90%igem Methanol gewaschen. Es werden 10,7 g N-Hexadecylmaleinimid erhalten, das von 46–49 °C schmilzt.

Zur Herstellung polymerer erfindungsgemässer Verbindungen geht man zweckmässigerweise aus von Polymeren mit funktionellen Seitengruppierungen, die umgesetzt werden mit geeigneten reaktionsfähigen Gruppen einer Verbindung, die die Struktur der angegebenen Formel I aufweist. Polymere mit Maleinsäure- oder Itaconsäureanhydrideinheiten können nach Houben-Weyl, Methoden der Organischen Chemie, Bd. XIV/2, Seite 736, mit hydroxylgruppenhaltigen Monomeren der Formel I zu den entsprechenden polymeren Halbestern umgesetzt werden (Verb. 14). Polymere, die in einer Seitenkette benachbart zu einer Carbonylgruppe eine aktivierte Methylengruppe aufweisen, können nach dem in DE-B-1 042 231 und DE-B-1 052 688 beschriebenen Verfahren mit aromatischen Aldehyden umgesetzt werden (Verb. 15). Die erfindungsgemässen Polymeren haben Molekulargewichte zwischen 5000 und 200 000.

Die erfindungsgemässen Verbindungen wirken vorzugsweise als Inhibitorfänger, wenn sie einem Kuppleremulgat, das Verunreinigungen an freiem Inhibitor enthält oder aus Gründen einer nicht ausreichenden Stabilität bei Lagerung oder bei höheren Temperaturen geringe Mengen an freiem Inhibitor unerwünscht in Freiheit setzt, direkt bei der Herstellung zugesetzt werden.

Wie bereits erwähnt, werden die erfindungsgemässen Verbindungen der Schicht des fotografischen Materials zugesetzt, die auch die nichtdiffundierende inhibitorfreisetzende Verbindung enthält. Die Einarbeitung erfolgt nach üblichen Methoden. Die Verbindungen der vorliegenden Erfindung können beispielsweise unter Ausnutzung vorhandener wasserlöslich machender Gruppen aus wässrig-alkalischen Lösungen in die Giesslösung eingebracht werden. Falls es sich um hydrophobe Verbindungen handelt, können sie auch in die jeweilige Schicht nach einem der bekannten Emulgierverfahren eingebracht werden. Solche Verfahren sind beispielsweise in den GB-A-791 219 und 1 099 414 bis 1 099 417, beschrieben. Weiterhin ist es möglich, wässrige Dispersionen der erfindungsgemässen Verbindungen herzustellen und den jeweiligen Giesslösungen zuzusetzen. Die Zugabe der erfindungsgemässen Verbindungen und der nichtdiffundierenden inhibitorfreisetzenden Verbindungen zu den Giesslösungen kann gleichzeitig oder nacheinander erfolgen. Die optimale Menge an erfindungsgemässer Verbindung kann innerhalb relativ weiter Grenzen variiert werden, wobei die günstigste Konzentration anhand einfacher Versuche festgestellt wird. Die Zugabe wird im allgemeinen so bemessen, dass die Menge an erfindungsgemässer Verbindung in der Schicht pro $m^2$ zwischen 1 mg und 1 g, vorzugsweise zwischen 2 mg und 100 mg, beträgt. Vorzugsweise wirksam sind Zusatzmengen, die zwischen 5 und 50 Gew.-% liegen, bezogen auf die im Emulgat vorhandene Menge an Inhibitor freisetzender Verbindung.

Bei dem erfindungsgemässen fotografischen Aufzeichnungsmaterial handelt es sich vorzugsweise um ein farbfotografisches Aufzeichnungsmaterial mit mehreren Silberhalogenidemulsionsschichten unterschiedlicher Spektralempfindlichkeit. So ist im allgemeinen eine der vorhandenen Silberhalogenidemulsionsschichten gegen blaues Licht empfindlich, eine weitere gegen grünes Licht und eine dritte gegen rotes Licht. Jeder dieser Schichten ist ein Farbkuppler zugeordnet zur Erzeugung eines Teilfarbbildes, dessen Farbe in der Regel komplementär ist zu der Farbe des Lichtes, gegen das die zugeordnete Silberhalogenidemulsionsschicht empfindlich ist. Gegebenenfalls können die genannten lichtempfindlichen Schichten auch aus 2 oder mehreren Teilschichten bestehen.

Das erfindungsgemässe farbfotografische Material enthält zugeordnet zu mindestens einer der genannten lichtempfindlichen Silberhalogenidemulsionsschichten eine nichtdiffundierende Verbindung, die unter den Bedingungen der fotografischen Entwicklung durch Einwirkung des Entwickleralkalis einen Entwicklungsinhibitor in Freiheit zu setzen vermag. Bei den so freigesetzten Entwicklungsinhibitoren handelt es sich um eine diffundierende heterocyclische Mercaptoverbindung. Vorzugsweise enthält die Mercaptoverbindung die Mercaptogruppe in einem heterocyclischen Ring an ein Kohlenstoffatom gebunden, das in Nachbarschaft zu einem Ringstickstoffatom steht, wie z.B. in 5-Mercapto-1-phenyltetrazol. Bei den nichtdiffundierenden, einen Entwicklungsinhibitor freisetzenden Verbindungen handelt es sich vorzugsweise um solche, bei denen der Entwicklungsinhibitor bildmässig als Folge einer Reaktion mit Farbentwickleroxidationsprodukten in Freiheit gesetzt wird. Dementsprechend erfolgt nach dieser bevorzugten Ausführungsform die Entwicklung mit einer Farbentwicklerverbindung, bei der es sich vorzugsweise um Derivate des p-Phenylendiamins mit mindestens einer primären Aminogruppe handelt.

Unter «Zuordnung» und «Zugeordnet» wird verstanden, dass die gegenseitige Anordnung von Silberhalogenidemulsion einerseits und Farbkuppler und/oder nichtdiffundierender Entwicklungsinhibitor freisetzender Verbindung und Verbindung gemäss der vorliegenden Erfindung anderseits von solcher Art ist, dass eine Wechselwirkung zwischen ihnen möglich ist, die eine bildmässige Übereinstimmung zwischen gebildetem Silberbild und bildmässiger Verteilung des gebildeten Farbstoffes bzw. gegebenenfalls des freigesetzten Entwicklungsinhibitors zulässt. Zweckmässigerweise wird hierbei die Zuordnung dadurch geschaffen, dass die genannten Verbindungen (Kuppler, Inhibitor freisetzende Verbindung und Verbindung gemäss der vorliegenden Erfindung) in der Silberhalogenidemulsionsschicht selbst enthalten sind. Es ist aber auch möglich, dass diese Verbindungen sich in einer zu der Silberhalogenidemulsionsschicht benachbarten, nicht lichtempfindlichen Schicht befinden. So kann beispielsweise der Farbkuppler in der lichtempfindlichen Silberhalogenidemulsionsschicht enthalten sein, während die Inhibitor freisetzende Verbindung, z.B. eine DIR-Verbindung, und die freien Inhibitor bindende Verbindung gemäss der vorliegenden Erfindung sich in einer zu der Silberhalogenidemulsionsschicht benachbarten nicht lichtempfindlichen Bindemittelschicht befinden. Die erfindungsgemässen Verbindungen sind auch wirksam, wenn sie allein für sich emulgiert fotografischen Schichten einverleibt sind, in denen aus homogen verteilten hydrophilen Schichtzusätzen bei Lagerung in unerwünschter Weise freier Inhibitor abgespalten wird.

Beispiele

Die Wirkungsweise der erfindungsgemässen Inhibitorfänger wird an folgendem farbfotografischem Material erläutert. Die angegebenen Mengen beziehen sich jeweils auf 1 m².

Eine rotempfindliche Schicht bestehend aus einer hochempfindlichen rotsensibilisierten Silberbromidjodidemulsion mit 10 Mol % Ag I (30%) und einer weniger empfindlichen rotsensibilisierten Silberbromidjodidemulsion mit 5 Mol % Ag I (70%) aus 4,0 g $AgNo_3$ mit 790 mg eines Blaugrünkupplers der Formel

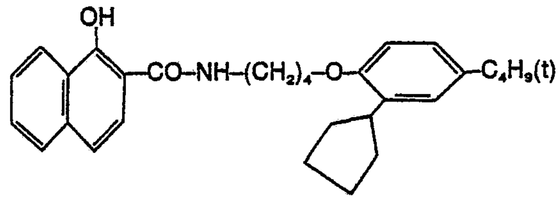

und 1,6 g Gelatine wird auf einer transparenten Filmunterlage aus Polyethylenterephthalat vergossen.

Darüber hinaus wird eine Schutzschicht aus 0,7 g Gelatine angebracht.

Das Material wird mit einem Sulfobetain-carbodiimid der Formel

$$CH_3{-}N{=}C{=}N{-}(CH_2)_3{-}\overset{\oplus}{N}(CH_3)_2{-}(CH_2)_4{-}SO_3^{\ominus}$$

gehärtet.

Bei einem Teil der Versuche wird der rotempfindlichen Schicht 25 mg eines DIR-Kupplers der Formel

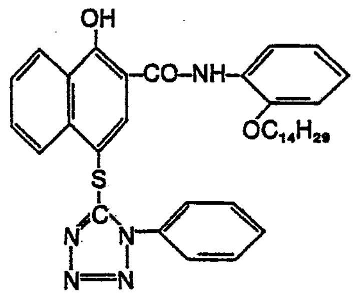

zugesetzt. Dieser DIR-Kuppler wird nach folgendem Rezept emulgiert:

Eine Lösung von 10 g DIR-Kuppler in 10 g Dibutylphthalat, 30 ml Essigsäureäthylester und 5 g Dimethylformamid wird unter intensivem Mischen in einer Mischsirene in eine Lösung aus 100 ml einer 5%igen wässrigen Gelatinelösung und 0,8 g eines Netzmittels, z.B. eines sulfierten Paraffinkohlenwasserstoffes, einemulgiert.

Im Falle des Zusatzes der erfindungsgemässen Inhibitorfänger und/oder des freien Inhibitors (1-Phenyl-5-mercaptotetrazol) zum DIR-Kuppler-Emulgat werden diese vor der Herstellung des Emulgates zusammen mit dem DIR-Kuppler gelöst.

Die Giessproben wurden hinter einem grauen Verlaufskeil mit rotem Licht belichtet und einer fotografischen Verarbeitung unterworfen, wie sie in «The British Journal of Photography», Juli 1974, Seiten 597 und 598, beschrieben ist.

Aus den Farbdichte-Kurven wird der Schleier, die Empfindlichkeit bei dem Kriterium 0,2-Dichteeinheiten über Schleier sowie die Gammawerte $\gamma_1$ und $\gamma_2$ bei 2-Gradationsabschnitten ($\gamma_1$ – vom Lichtempfindlichkeitspunkt bis zu einem um 0,8 logI·t-Einheiten höheren Belichtungswert; $\gamma_2$ –

vom Endpunkt von $\gamma_1$ bis zu einem um weitere 0,8 logI·t-Einheiten höheren Belichtungswert) angegeben. Die Ergebnisse sind aus der folgenden Tabelle ersichtlich.

Tabelle

| Probe | DIR-Kuppler | freier Inhibitor | Inhibitorfänger | Nr. | Empf. | $\gamma_1/\gamma_2$ | Schleier |
|---|---|---|---|---|---|---|---|
| 1 | – | – | – | | 35,7 | 1,22/0,50 | 0,22 |
| 2 | 25 mg | – | – | | 32,7 | 0,50/0,55 | 0,19 |
| 3 | 25 mg | – | 5 mg | 1 | 32,6 | 0,51/0,56 | 0,19 |
| 4 | 25 mg | 0,25 mg | – | | 31,7 | 0,44/0,48 | 0,16 |
| 5 | 25 mg | 0,25 mg | 5 mg | 1 | 32,5 | 0,51/0,54 | 0,19 |
| 6 | 25 mg | 0,25 mg | 5 mg | 2 | 32,8 | 0,49/0,54 | 0,18 |
| 7 | 25 mg | 0,25 mg | 5 mg | 3 | 32,7 | 0,52/0,54 | 0,20 |
| 8 | 25 mg | 0,25 mg | 5 mg | 4 | 32,6 | 0,50/0,56 | 0,19 |
| 9 | 25 mg | 0,25 mg | 5 mg | 5 | 32,5 | 0,49/0,53 | 0,17 |
| 10 | 25 mg | 0,25 mg | 5 mg | 7 | 33,0 | 0,53/0,56 | 0,20 |
| 11 | 25 mg | – | 5 mg | 2 | 32,7 | 0,50/0,52 | 0,19 |
| 12 | 25 mg | – | 5 mg | 3 | 32,7 | 0,53/0,52 | 0,19 |
| 13 | 25 mg | – | 5 mg | 4 | 32,5 | 0,52/0,56 | 0,17 |
| 14 | 25 mg | – | 5 mg | 5 | 32,5 | 0,50/0,51 | 0,18 |
| 15 | 25 mg | – | 5 mg | 7 | 32,9 | 0,51/0,57 | 0,20 |

Durch Zusatz von DIR-Kuppler wird Gradation ($\gamma_1$), Empfindlichkeit und Schleier erniedrigt (Probe 1 und 2). Durch Verunreinigung des DIR-Kupplers mit 1% freiem Inhibitor (1-Phenyl-5-mercaptotetrazol) bei Probe 4 wird gegenüber Probe 2 Empfindlichkeit und Gradation unerwünscht gedrückt. Der Zusatz der Inhibitorfänger zu dem mit freiem Inhibitor verunreinigten DIR-Kuppler-Emulgat bei den Proben 5 bis 10 verhindert die Hemmwirkung des freien Inhibitors (Probe 4), so dass die sensitometrischen Daten der Probe 2, die keine Verunreinigung an freien Inhibitor enthält, wieder erhalten werden. Die Inhibitorfänger selbst bewirken praktisch keine Beeinflussung der sensitometrischen Daten, wenn keine Verunreinigung mit freiem Inhibitor vorliegt, wie die Proben 3 und 11 bis 15 zeigen.

**Patentansprüche**

1. Fotografisches Aufzeichnungsmaterial mit mindestens einer lichtempfindlichen Silberhalogenidemulsionsschicht und mindestens einer Schicht, die eine nichtdiffundierende Verbindung enthält, die unter den Bedingungen der alkalischen Entwicklung eine diffundierende heterocyclische Mercaptoverbindung als Entwicklungsinhibitor in Freiheit zu setzen vermag, dadurch gekennzeichnet, dass die gleiche Schicht, die die nichtdiffundierende, einen Entwicklungsinhibitor freisetzende Verbindung enthält, eine weitere Verbindung enthält, die bei pH-Werten kleiner als 7, nicht aber oder nur sehr viel weniger bei pH-Werten zwischen 9 und 13, freien Entwicklungsinhibitor zu binden vermag.

2. Aufzeichnungsmaterial nach Anspruch 1, dadurch gekennzeichnet, dass die weitere Verbindung der folgenden allgemeinen Formel I entspricht:

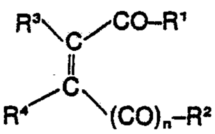

I

worin bedeuten

$R^3$ und $R^2$ (gleich oder verschieden) Wasserstoff, Alkyl, Aryl, Hydroxyl, Alkoxy, —NR-Alkyl oder —NR-Aryl, wobei R für Wasserstoff oder Alkyl steht, oder beide zusammen den Rest zur Vervollständigung eines 5- oder 6gliedrigen carbocyclischen oder heterocyclischen Ringes, der einen ankondensierten Ring aufweisen kann,

$R^3$ und $R^4$ (gleich oder verschieden) Wasserstoff, Alkyl mit bis zu 18 C-Atomen, Aryl, Halogen, Alkoxycarbonyl oder Cyan

n = 0 oder 1.

3. Aufzeichnungsmaterial nach Anspruch 2, dadurch gekennzeichnet, dass die weitere Verbindung in wenigstens einem der Substituenten $R^1$, $R^2$, $R^3$ und $R^4$ einen diffusionsfestmachenden Rest enthält und in nichtdiffundierender Form in der Schicht enthalten ist.

4. Aufzeichnungsmaterial nach Anspruch 2, dadurch gekennzeichnet, dass $R^1$ und $R^2$ zusammen einen p-Chinonring oder einen am Stickstoffatom gegebenenfalls substituierten Maleinimidring vervollständigen (n = 1).

5. Aufzeichnungsmaterial nach Anspruch 1, dadurch gekennzeichnet, dass die nichtdiffundierende, einen Entwicklungsinhibitor freisetzende Verbindung und die weitere Verbindung in einer lichtempfindlichen Silberhalogenidemulsionsschicht enthalten sind.

6. Aufzeichnungsmaterial nach einem der Ansprüche 1 bis 5, dadurch gekennzeichnet, dass es mehrere Silberhalogenidemulsionsschichten unterschiedlicher Spektralempfindlichkeit und diesen zugeordnete, nichtdiffundierende Farbkuppler enthält.

**Claims**

1. Photographic recording material having at least one light-sensitive silver halide emulsion layer and at least one layer containing a non-diffusible compound which is capable of releasing a diffusible heterocyclic mercapto compound as development inhibitor under the conditions of alkaline development, characterised in that the same layer which contains the non-diffusible compound releasing a development inhibitor contains another compound which is capable of binding free development inhibitor at pH values below 7 but is not capable of doing so, or only to a much less extent, at pH values between 9 and 13.

2. Recording material according to claim 1, characterised in that the other compound corresponds to the following general formula I

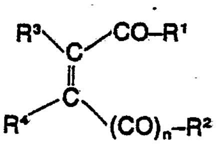

I

wherein

$R^1$ and $R^2$ (which are the same or different) denote hydrogen, alkyl, aryl, hydroxyl, alkoxy, —NR-alkyl or —NR-aryl, wherein R represents hydrogen or alkyl, or both together denote the radical to complete a 5- or 6-membered carbocyclic or heterocyclic ring which can contain a fused-on ring,

$R^3$ and $R^4$ (which are the same or different) denote hydrogen, alkyl with up to 18 C atoms, aryl, halogen, alkoxycarbonyl or cyano, and

$n = 0$ or 1.

3. Recording material according to claim 2, characterised in that the other compound contains a diffusion resistance conferring radical in at least one of the substituents $R^1$, $R^2$, $R^3$ and $R^4$ and is contained in the layer in a non-diffusible form.

4. Recording material according to claim 2, characterised in that $R^1$ and $R^2$ together complete a p-quinone ring or a maleic imide ring ($n = 1$) which is optionally substituted at the nitrogen atom.

5. Recording material according to claim 1, characterised in that the non-diffusible compound which releases a development inhibitor and the other compound are contained in a light-sensitive silver halide emulsion layer.

6. Recording material according to one of claims 1 to 5, characterised in that it contains several silver halide emulsion layers of differing spectral sensitivity and non-diffusible colour couplers associated therewith.

**Revendications**

1. Elément d'enregistrement photographique comportant au moins une couche d'émulsion photosensible à l'halogénure d'argent et au moins une couche contenant un composé non diffusible qui, dans les conditions du développement alcalin, peut libérer, comme inhibiteur de développement, un mercapto-composé hétérocyclique diffusible, caractérisé en ce que la même couche qui contient le composé non diffusible libérant un inhibiteur de développement, contient un composé supplémentaire pouvant fixer un inhibiteur libre de développement à des pH inférieurs à 7, mais non ou alors beaucoup moins à des pH se situant entre 9 et 13.

2. Elément d'enregistrement suivant la revendication 1, caractérisé en ce que le composé supplémentaire répond à la formule générale I ci-après:

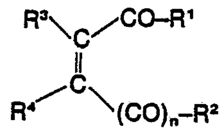

I

dans laquelle

$R^1$ et $R^2$ (identiques ou différents) représentent chacun un atome d'hydrogène, un groupe alkyle, un groupe aryle, un groupe hydroxy, un groupe alcoxy, un groupe —NR-alkyle ou un groupe —NR-aryle, R représentant un atome d'hydrogène ou un groupe alkyle, ou les deux radicaux ensemble représentent le radical destiné à compléter un noyau carbocyclique ou hétérocyclique pentagonal ou hexagonal pouvant comporter un noyau condensé,

$R^3$ et $R^4$ (identiques ou différents) représentent chacun un atome d'hydrogène, un groupe alkyle contenant jusqu'à 18 atomes de carbone, un groupe aryle, un atome d'halogène, un groupe alcoxycarbonyle ou un groupe cyano,

$n = 0$ ou 1.

3. Elément d'enregistrement suivant la revendication 2, caractérisé en ce que, dans au moins un des substituants $R^1$, $R^2$, $R^3$ et $R^4$, le composé supplémentaire contient un radical conférant une résistance à la diffusion, ce composé étant contenu dans la couche sous une forme non diffusible.

4. Elément d'enregistrement suivant la revendication 2, caractérisé en ce que $R^1$ et $R^2$ ensemble complètent un noyau p-quinone ou un noyau maléimido éventuellement substitué sur l'atome d'azote ($n = 1$).

5. Elément d'enregistrement suivant la revendication 1, caractérisé en ce que le composé non diffusible libérant un inhibiteur de développement et le composé supplémentaire sont contenus dans une couche d'émulsion photosensible à l'halogénure d'argent.

6. Elément d'enregistrement suivant l'une quelconque des revendications 1 à 5, caractérisé en ce qu'il comporte plusieurs couches d'émulsions aux halogénures d'argent d'une sensibilité spectrale différente, ainsi que des copulants chromogènes non diffusibles attribués à ces couches.